# EUROPÄISCHE PATENTANMELDUNG

(11) **EP 2 241 353 A2**
(43) Veröffentlichungstag der Anmeldung: **20.10.2010**
(21) Anmeldenummer: 09176845.7
(22) Anmeldetag: 24.11.2009
(51) Int. Cl.: A61Q 19/00, A61K 8/04, A61K 8/893

(54) **Schäumende Emulsionen mit Polymeren**

(30) Priorität: 27.11.2008 DE 102008059443
(71) Anmelder: Henkel AG & Co. KGaA, 40589 Düsseldorf (DE)
(72) Erfinder: Janßen, Frank, 41470, Neuss (DE); Hentrey, Stefanie, 20259, Hamburg (DE)

(57) **Zusammenfassung**

Gegenstand der Erfindung ist ein System aus einem manuell anwendbaren Schaumspender und einer kosmetischen Pflegeemulsion, die
(a) mindestens einen hautpflegenden Ölkörper,
(b) ein Tensidgemsich aus
(i) mindestens einem milden anionischen Tensid und
(ii) mindestens einem milden amphoteren und/oder zwitterionischen Tensid und
(c) einen Silikonemulgator mit einem HLB-Wert im Bereich von 4 bis 6,

enthält.

## Beschreibung

Die Erfindung betrifft ein System aus einem manuell anwendbaren Schaumspender und einer kosmetischen Pflegeemulsionen auf der Basis einer speziellen Tensidkombination und eines speziellen Emulgators, sowie die Verwendung des Systems zur Pflege von Keratinsubstanzen. Schäumende Emulsionen sind im Stand der Technik im Prinzip bekannt, doch weisen diese oftmals ungünstige Eigenschaften wie schlechte Verteilbarkeit oder ein stumpfes Hautgefühl nach der Anwendung auf.

Daher wurden in jüngster Zeit tensidhaltige Emulsionen entwickelt, die dank eines speziellen Pumpmechanismus mit Luft verschäumt werden, und sich als Schaum leichter und gleichmäßiger auf der Haut verteilen lassen.

Bei der Weiterentwicklung der verschäumbaren Emulsionen wurde jedoch festgestellt, dass der Schaum nicht fest genug war und schnell brach, wodurch auf der Haut nach der Anwendung ein wässriges und unangenehmes Gefühl hinterlassen wurde.

Um diese Nachteile zu beseitigen wurde in der EP 1 496 852 vorgeschlagen, Polyolpoly-12-Hydroxystearate als Emulgatoren mit Tensiden, Ölkörpern, Lichtschutzfiltern und Wasser zu einer Emulsion in einem manuell betätigbaren Pumpspender zu verschäumen.

Auch diese Systeme konnten, insbesondere für den Zweck einer kosmetischen Hautpflegeemulsion, hinsichtlich Ihrer Schaumqualität und Hautpflegeeigenschaften nicht zufriedenstellen, denn der entstehende Schaum war nicht dicht genug und wies eine rasch eintretende Drainage auf.

Des Weiteren führte der Schaum nach der Applikation zu einem stark klebrigen und stumpfen Hautgefühlt, was dem Verbraucher nicht das gewünschte Gefühl von Pflege und Regeneration der Haut vermittelte.

Aufgabe der vorliegenden Erfindung war es daher ein System zur Verfügung zu stellen, das die vorgenannten Nachteile nicht aufweist. Insbesondere sollte eine in einem manuell betätigbaren Schaumspender konfektionierte Pflegeemulsion zur Verfügung gestellt werden, deren Schaum feinporig und stabil ist und dem Verbraucher beim Verteilen ein besonders leichtes und gleichzeitig reichhaltiges Pflegeerlebnis vermittelt.

Überraschend wurde nun gefunden, dass sich diese Ziele erreichen lassen, wenn man eine Emulsion auf der Basis einer speziellen Tensidkombination und eines speziellen Emulgators in einem manuell anwendbaren Schaumspender konfektioniert.

Außergewöhnlich an der vorliegenden Erfindung ist, dass der erfindungsgemäße Emulgator zwingend ein Silikoemulgator eines bestimmten HLB-Werts ist. Silikone werden üblicherweise chemischen Systemen hinzugefügt, um das Schäumen zu verhindern, daher war es nicht erwartet den erfindungsgemäßen Effekt mit einem Silikonemulgator zu erzielen.

Gegenstand der Erfindung sind daher Systeme gemäß Anspruch 1.

Erfindungsgemäße Systeme liefern einen besonders feinporigen, dichten, stabilen und cremigen Schaum, der sich einfach auf der Haut verteilen lässt und sowohl während, als auch nach der Produktabsorption ein konstant gleitendes und weiches Pflegegefühl hinterlässt.

Unter dem Begriff "Emulsion" wird erfindungsgemäß auf der einen Seite eine homogene Emulsion verstanden, in der die Wasser- und die Ölphase über einen sehr langen Zeitraum stabil ineinander emulgiert vorliegen. Gleichzeitig ist es erfindungsgemäß aber auch möglich, dass die "Emulsion" erst unmittelbar vor dem Aufschäumen (das bedeutet unmittelbar vor der Betätigung des Schaumspenders) hergestellt wird, indem die Öl- und die Wasserphase, die getrennt voneinander vorliegen, durch kräftiges Schütteln kurzzeitig ineinander emulgiert werden.

Durch beide Ausführungsformen werden die erfindungsgemäßen Ziele erreicht.

Ein zweiter Gegenstand der Erfindung ist die Verwendung eines Silikonemulgators eines HLB-Werts von 4 bis 6 in kosmetischen Pflegeemulsionen, die in einem manuell anwendbaren Schaumspender konfektioniert sind.

Ein dritter Gegenstand der Erfindung ist die Verwendung des erfindungsgemäßen Systems zur Pflege von Keratinfasern, insbesondere der menschlichen Haut.

Die Emulgatorauswahl erfolgte sehr behutsam, denn es galt die vorgenannten Probleme zu beseitigen und bekannte Rezepturen zu verbessern. Der oder die Emulgator(en) sollten optimalerweise auch zu den pflegenden Eigenschaften der Pflegeemulsionen beitragen, ohne dass negative Wechselwirkungen mit anderen Bestandteilen oder Hautunverträglichkeiten auftraten.

Es wurde festgestellt, dass sich für diesen Zweck Emulgatoren eignen, die einen HLB-Wert im Bereich von 2 bis 8, bevorzugt im Bereich von 3 bis 7 und insbesondere im Bereich von 4 bis 6 aufweisen. Emulgatoren mit einem HLB-Wert von 4,5, 4,6, 4,7, 4,8, 4,9, 5,0, 5,1, 5,2, 5,3, 5,4 oder 5,5 sind dabei besonders bevorzugt; außerordentlich bevorzugt sind Emulgatoren mit einem HLB-Wert von 4,8, 4,9, 5,0, 5,1 oder 5,2.

Ein Emulgator, der sich erfindungsgemäß zur Erfüllung der vorgenannten Anforderungen in hohem Maße eignet, ist ein Silikonemulgator eines HLB-Werts im Bereich von 4 bis 6.

Bei diesem handelt es sich in einer bevorzugten Ausführungsform um einen Organopolysiloxan-Polyoxyalkylen-Emulgator, der - bezogen auf das Gesamtgewicht der Pflegeemulsionen - in einer Menge von 0,2 bis 20 Gew.-%, bevorzugt 0,5 bis 15 Gew.-%, mehr bevorzugt 1,0 bis 12

Gew.-% und insbesondere 1,5 bis 10 Gew.-% eingesetzt wird.

Erfindungsgemäß geeignete Organopolysiloxan-Polyoxyalkylen-Emulgatoren entsprechen der allgemeinen Formel (1), 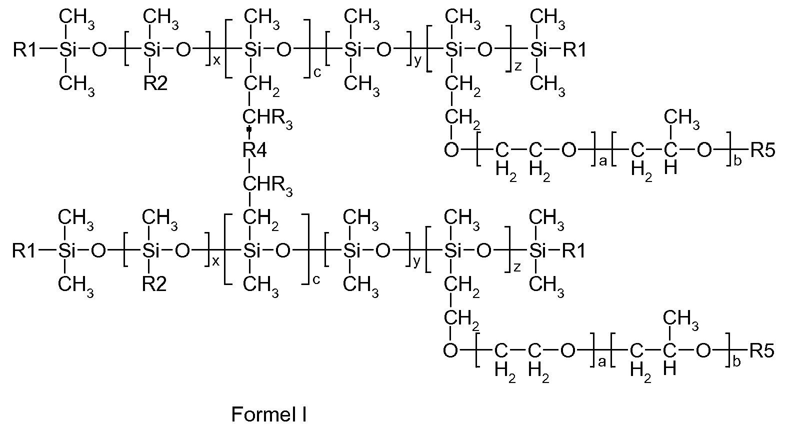 in der
die Reste R1 unabhängig voneinander aliphatische Reste mit 1 bis 25 Kohlenstoffatomen,
die Reste R2 unabhängig voneinander aliphatische Reste mit 2 bis 25 Kohlenstoffatomen,
die Reste R3 unabhängig voneinander Wasserstoff oder aliphatische Reste mit 1 bis 3 Kohlenstoffatomen, der Rest R4 eine aorganische oder Organosiloxan-Gruppe ist, die keine hydrolysierbaren Bindungen enthält, die Reste R5 unabhängig voneinander Endgruppen sind, die mit den Bestandteilen, die den Emulgator stabilisieren sollen, nicht nachteilig reagieren und die Bildung des Organopolysiloxan-Polyoxyalkylens nicht stören, stehen, und
x für einen Wert von 1 bis 100,
y für einen Wert von 0 bis 600,
z für einen Wert von 1 bis 100 steht, wobei x+y+z mindestens 30 ist,
a für einen Wert von 4 bis 40,
b für einen Wert von 0 bis 40 und
c für einen Wert von 0 bis 5 steht, wobei das Verhältnis von a : b 20 : 80 bis 100 : 0 ist. Erfindungsgemäß besonders bevorzugt sind Organopolysiloxan-Polyoxyalkylen-Emulgatoren, in denen R1 für Methyl, R2 für C₈-C₁₈-Alkyl, R3 für Wasserstoff, R4 für -(CH₃)₂-Si-O-Si-(CH₃)₂-, R5 für Wasserstoff, x für 5 bis 60, y für 0 bis 150, z für 1 bis 15, a für 10 bis 30, b für 10 bis 30 und c für 0 oder 1 steht. Insbesondere bevorzugt sind solche Organopolysiloxan-Polyoxyalkylen-Emulgatoren, in denen R2 für C16-Alkyl, x für 5 bis 50, y für 25 bis 150, z für 1 bis 15 und c für 0 steht und das Verhältnis von a : b bei 40 : 60 bis 70 : 30 liegt.

Ein Beispiel für einen solchen insbesondere bevorzugten Silikonemulgator ist der unter der INCI-Bezeichnung "Cetyl PEG/PPG-10/1-Dimethicone" (vormals: Cetyl Dimethicone Copolyol) und dem Handelsnamen "Abil EM 90" von der Firma Goldschmidt vertriebene W/O-Emulgator. Abil EM 90 weist nach Herstellerangaben einen HLB-Wert von etwa 5 auf.

Für die erfindungsgemäßen Systeme ist es ebenfalls erforderlich, dass sie die kosmetischen Eigenschaften der Haut wie Glätte, Weichheit und Feuchtigkeit verbessern. Um diesen Anforderungen nachzukommen enthalten die Emulsionen des erfindungsgemäßen Systems bevorzugt einen Wirkstoff, der der Haut mit Feuchtigkeit spendet.

Bei diesem handelt es sich beispielsweise um Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Sorbitol, Milchsäure, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Besonders bevorzugt sind Glycerin und Sorbitol und insbesondere Glycerin.

Die Feuchthaltemittel werden den Emulsionen des erfindungsgemäßen Systems - bezogen auf deren Gesamtgewicht - in einer Menge von 0,1 bis 15 Gew.-%, bevorzugt von 0,25 bis 10 Gew.-% und insbesondere von 0,5 bis 5 Gew.-% zugesetzt.

Zur Erhöhung der Schaumbildung und -stabilisierung enthalten die erfindungsgemäßen Pflegeemulsionen zwingend anionische sowie amphotere und/oder zwitterionische Tenside. Auch bei den Tensiden musste eine behutsame Auswahl stattfinden, denn einerseits sollten die Tenside besonders mild sein, um eine hautschonende Behandlung ohne das Auftreten von Hautirritationen zu gewährleisten, und andererseits sollten die Tenside eine ausreichende Schaumbildung aufweisen, um für die erfindungsgemäße Applikation in einem manuell betätigbaren Schaumspender geeignet zu sein.

Als erfindungsgemäß geeignete Tensidkombination zur Erfüllung der vorgenannten Erfordernisse haben sich milde anionische und milde amphotere/zwitterionische Tenside herausgestellt, die ausgewählt sind aus einem oder mehreren Tensiden der Gruppe (iii) der Acylaminosäuren, alpha-Olefinsulfonaten, ethoxylierten Alkylschwefelsäurestern, Sulfobernsteinsäureestern, Ethercarbonsäuren und/oder den Salzen dieser Verbindungen, sowie aus einem oder mehreren Tensiden der Gruppe (iv) der Alkylbetaine, Sulfobetaine, Alkylamidoalkylbetaine, Aminopropionate, Aminoglycinate und/oder Imidazoliniumbetaine und/oder den Salzen dieser Verbindungen.

Unter den erfindungsgemäß geeigneten anionischen Tensiden (iii) sind Verbindungen zu verstehen, die unter den Natrium-, Kalium- und Ammonium- sowie der Mono-, Di- und Trialkanolammoniumsalzen mit 2 bis 4 C-Atomen in der Alkanolgruppe, der
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Acylglutamate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alpha-Olefinsulfonate mit 8 bis 24 C-Atomen,
- Alkylsulfate und Alkylpolyglykolethersulfate der Formel R-O(CH₂CH₂O)ₓ-OSO₃H, in der R eine bevorzugt lineare Alkylgruppe mit 8 bis 30 C-Atomen und x = 0 oder 1 bis 12 ausgewählt sind.

Besonders geeignete anionische Tenside (iii) in den erfindungsgemäßen Pflegeemulsionen sind aufgrund ihrer ausgezeichneten Milde und Schäumbarkeit die Sulfobernsteinsäuremonoester-Salze von Fettalkoholen und Fettalkoholpolyglycolethern der allgemeinen Formel

R1-O-(CH₂-CH₂-O)ₙ-CO-CH₂-CH(SO₃⁻)-COO⁻ M₂⁺,

in der R1 eine Alkylgruppe mit 12 bis 18 Kohlenstoffatomen, n einen Mittelwert von 0-6 aufweist und M ein Alkalikation, bevorzugt Natrium, ist. Insbesondere geeignet sind Sulfobernsteinsäuremonoester-Salze der Formel

R1-O-(CH₂-CH₂-O)ₙ-CO-CH₂-CH(SO₃Na)-COONa,

in der R1 eine Alkylgruppe mit 12 bis 16 Kohlenstoffatomen und n einen Mittelwert von 1-6 aufweist. Solche Produkte mit n=3 sind unter der Handelsbezeichnung Texapon^{®} SB 30 von der Firma Cognis im Handel erhältlich.
Das oder die anionische(n) Tensid(e) wird (werden) in den erfindungsgemäßen Pflegeemulsionen - bezogen auf ihr Gesamtgewicht - bevorzugt in Mengen von 0,1 bis 20 Gew.-%, bevorzugt von 0,2 bis 15 Gew.-% und insbesondere in Mengen von 0,5 bis 9 Gew.-% eingesetzt.

Erfindungsgemäß geeignete zwitterionische Tenside sind die sogenannten Betaine wie die Alkylbetaine, N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Ein erfindungsgemäß bevorzugtes zwitterionisches Tensid ist das unter der INCI-Bezeichnung Cocamidopropyl Betaine bekannte Fettsäureamid-Derivat.

Beispiele für erfindungsgemäß geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und

Alkylaminoessigsäuren mit jeweils etwa 8 bis 24 C-Atomen in der Alkylgruppe.

Erfindungsgemäß bevorzugte ampholytische Tenside sind das N-Kokosalkylaminopropionat, das Kokosacylaminoethylaminopropionat und das C₁₂ - C₁₈ - Acylsarcosin.

Besonders geeignete amphotere und/oder zwitterionische Tenside (iv) in den erfindungsgemäßen Pflegeemulsionen sind aufgrund ihrer ausgezeichneten Milde und Schäumbarkeit Ampho- und Betaintenside, die eine Alkyl- bzw. Acylgruppe mit 8 bis 10 Kohlenstoffatomen aufweisen, insbesondere Betaintenside der Formel

R2CONH(CH₂)ₘ-N⁺(CH₃)₂-COO⁻,

in der R2CO eine von Capryl- und Caprinsäure abgeleitete Gruppe und m=3 ist. Ein solches Tensid ist als besonders haut- und schleimhautfreundliches sowie reizarmes Betaintensid bekannt, und unter der Bezeichnung Tego Betain 810 von der Firma Goldschmidt im Handel erhältlich.

Das oder die amphotere(n) und/oder zwitterionische(n) Tensid(e) wird (werden) in den erfindungsgemäßen Pflegeemulsionen - bezogen auf ihr Gesamtgewicht - bevorzugt in Mengen von 0,1 bis 20 Gew.-%, bevorzugt von 0,2 bis 15 Gew.-% und insbesondere in Mengen von 0,5 bis 9 Gew.-% eingesetzt.

In einer weiteren bevorzugten Ausführungsform der Erfindung werden den Pflegeemulsionen des Systems zusätzlich zu den vorgenannten zwingenden Komponenten weiterhin Stoffe zur Erhöhung der Emulsions- und Schaumstabilität zugesetzt.

Es wurde gefunden, dass sich eine besonders gute Schaumstabilisierung erzielen lässt, wenn eine Kombination verschiedener Polymere verwendet wurde.

Als insbesondere geeignet für die erfindungsgemäßen Pflegeemulsionen hat sich die Kombination aus mindestens zwei Polymeren erwiesen, von denen eines natürlichen und das andere synthetischen Ursprungs ist.

Erfindungsgemäß geeignete Polymere natürlichen Ursprungs basieren auf Xanthan Gum, Guar Gum, Alginaten und/oder Carrageenaten. Als besonders bevorzugt haben sich Polymere herausgestellt, die sich aus Alginaten herstellen lassen, wobei insbesondere veresterte Alginate erfindungsgemäß geeignet sind. Neben der schaum- und emulsionsstabilisierenden Wirkung haben diese Verbindungen den Vorteil, dass sie dazu beitragen können den Wasserverlust in der Haut reduzieren, wodurch sie die feuchtigkeitsspendende Wirkung der erfindungsgemäßen Pflegeemulsionen zusätzlich unterstützen.

Besonders geeignete Alginatester werden aus Alginaten hergestellt, die mit einem oder mehreren C₁-C₆-Alkoholen, welche eine oder mehrere Hydroxygruppen tragen können, verestert werden. Insbesondere geeignet sind Propylenglycolalginate, die durch die Umsetzung eines Alginats mit Propylenoxid bei hohen Temperaturen entstehen. Solche veresterten Alginate sind beispielsweise von der Firma FMC Biopolymers unter der Bezeichnung "Protanal Ester" im Handel erhältlich. Protanal Ester^{®} CF ist für die erfindungsgemäße Pflegeemulsion besonders bevorzugt, denn er weist in wässrigen, alkoholischen oder wässrig alkoholischen Lösungen eine niedrige Viskosität auf, was sich auf die leichte Verschäumbarkeit der Pflegeemulsionen positiv auswirkt.

Die Polymere natürlichen Ursprungs werden in den erfindungsgemäßen Pflegeemulsionen - bezogen auf deren Gesamtgewicht - in Mengen von 0,01 bis 10 Gew.-%, bevorzugt von 0,05 bis 5 Gew.-% und insbesondere in Mengen von 0,1 bis 2 Gew.-% eingesetzt.

Besonders bevorzugte anionische Homo- und Copolymere sind hierbei vernetzte Polyacrylsäuren, die teilweise oder vollständig neutralisiert sind. Solche Polymere sind zum Beispiel die Handelsprodukte der Carbopol^{®}-Serie, wie Carbopol^{®} 934, 940, 941, Carbopol^{®} Ultrez 10, Carbopol^{®} ETD 2001 und 2050 sowie die Produkte Tego Carbomer 140 und 141 und Synthalen^{®} L.

Die Polyacrylsäuren werden in den erfindungsgemäßen Pflegeemulsionen - bezogen auf deren Gesamtgewicht - bevorzugt in einer Menge von 0,01 bis 4 Gew.-%, besonders bevorzugt von 0,02 bis 2 Gew.-% und außerordentlich bevorzugt in einer Menge von 0,03 bis 1 Gew.-% eingesetzt.

Weitere erfindungsgemäß bevorzugte synthetische Polymere sind Polyacryloyldimethyltaurate, wie beispielsweise die vernetzten Acryloyldimethyltaurat-Acrylamid-Copolymere mit dem Handelsnamen Sepigel^{®} 305 oder Simulgel^{®} 600 der Firma Seppic, die Homopolymere der 2-Acrylamido-2-methylpropansulfonsäure (Acryloyldimethyltaurin), die gegebenenfalls vernetzt und/oder neutralisiert sein können, wie beispielsweise Poly(2-acrylamido-2-methylpropansulfonsäure), die von der Firma Hoechst unter dem Handelsnamen Hostacerin^{®} AMPS oder von der Firma Seppic unter dem Handelsnamen Simulgel^{®} 800 vertrieben wird, und

Copolymere der 2-Acrylamido-2-methylpropansulfonsäure und Hydroxyethylacrylate, wie beispielsweise die unter den Handelnamen Sepinov^{®} EMT 10 oder Simulgel^{®} NS von der Firma Seppic vertriebenen Handelsprodukte.

Die Polyacryloyldimethyltaurate werden in den erfindungsgemäßen Pflegeemulsionen - bezogen auf deren Gesamtgewicht - bevorzugt in einer Menge von 0,01 bis 5 Gew.-%, besonders bevorzugt von 0,02 bis 2 Gew.-% und außerordentlich bevorzugt in einer Menge von 0,05 bis 1 Gew.-% eingesetzt.

Als besonders geeignet für die Herstellung der erfindungsgemäßen Pflegeemulsionen hat sich die Kombination einer Polyacrylsäure mit einem Polyacryloyldimethyltaurat erwiesen, wobei die beste Schaum- und Emulsionsstabilisierung durch die Kombination der beiden Polymere Tego Carbomer^{®} 140 und Sepinov^{®} EMT 10 erzielt werden konnte.

In einer besonders bevorzugten Ausführungsform der Erfindung eignet sich als Polymerkombination zur optimalen Schaum- und Emulsionsstabilisierung insbesondere die Kombination eines Alginatesters mit einer Polyacrylsäure und einem Polyacryloyldimethyltaurat, und insbesondere die Kombination der Handelsprodukte Protanal Ester^{®} CF, Tego Carbomer^{®} 140 und Sepinov^{®} EMT 10.

In einer besonders bevorzugten Ausführungsform der Erfindung eignet sich als Polymerkombination zur optimalen Schaum- und Emulsionsstabilisierung insbesondere die Kombination eines Alginatesters mit einer Polyacrylsäure und einem Polyacrylamid, und insbesondere die Kombination der Handelsprodukte Protanal Ester^{®} CF, Tego Carbomer^{®} 140 und Sepinov^{®} EMT 10.

Die zur Herstellung der Pflegemulsionen in den erfindungsgemäßen Systemen ebenfalls zwingend erforderliche Ölkomponente muss Hautpflegende Eigenschaften aufweisen, mit den vorgenannten, zwingenden Komponenten leicht emulgierbar sein und die Emulsionsstabilität nicht negativ beeinflussen. Des weiteren darf die Ölkomponente die Schaumentwicklung sowie die Sensorik des Schaums nicht beeinträchtigen. Unter diesen Voraussetzungen sind sie folgenden natürlichen und synthetischen Ölkomponenten und/oder Fettstoffe erfindungsgemäß geeignet: - natürliche (pflanzliche) Öle: dabei handelt es sich üblicherweise um Triglyceride und

Mischungen von Triglyceriden. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Kakaoabutter und Shea-Butter. Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).

Als synthetische Öle kommen auch Silikonverbindungen wie Cyclomethicone, Dimethicone und Amodimethicone in Betracht.

Als Ölkomponente kann weiterhin ein Dialkylether dienen.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyln-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Cetiol^{®} OE erhältlich ist.

Unter erfindungsgemäß geeigneten Fettstoffen sind Fettsäuren, Fettalkohole sowie natürliche und synthetische Wachse zu verstehen, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®}871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

### Weitere Fettstoffe sind beispielsweise

- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD, und
- Ester der Benzoesäure mit linearen und/oder verzweigten C₆-C₂₂-Alkoholen, wie beispielsweise das unter der Handelsbezeichnung Finsolv^{®} TN vertriebene C₁₂-C₁₅-Alkylbenzoat.

Die Cremigkeit und die Feuchtigkeit- und Glätte-spendenden Eigenschaften werden besonders positiv beeinflusst, wenn als Ölkomponenten Dialkylether, Dialkylcarbonate und Benzoesäureester eingesetzt werden. Erfindungsgemäß besonders geeignet war eine Kombination aus Dicaprylylether (z.B. Cetiol^{®} OE), Ethylhexyl Palmitate (z.B. Cegesoft^{®} C24) und C₁₂-C₁₅-Alkylbenzoat (z.B. Finsolv^{®} TN).

Die Gesamtmenge der vorgenannten Öl- und Fettkomponenten in den erfindungsgemäßen Pflegeemulsionen beträgt - bezogen auf das Gesamtgewicht der Pflegeemulsionen - 1 - 25 Gew.-%, bevorzugt 3 bis 20 Gew.-%, mehr bevorzugt 5 bis 15 Gew.-% und insbesondere 6 bis 10 Gew.-%.

Falls die erfindungsgemäßen Pflegeemulsionen neben den vorgenannten Öl- und

Fettkomponenten weitere hydrophobe Wirkstoffe, wie insbesondere Lichtschutzfilter, enthalten soll, ist es besonders bevorzugt, dass die Gesamtmenge der emulgierten Ölphase, einschließlich der vorgenannten Öl- und Fettkomponenten und weiterer hydrophober Wirkstoffe in den erfindungsgemäßen Pflegeemulsionen - bezogen auf das Gesamtgewicht der Pflegeemulsionen - 1 - 25 Gew.-%, bevorzugt 3 bis 20 Gew.-%, mehr bevorzugt 5 bis 15 Gew.-% und insbesondere 6 bis 10 Gew.-% beträgt.

Besonders bevorzugte erfindungsgemäße Pflegeemulsionen sind **dadurch gekennzeichnet, dass** die vorgenannten Öl- und Fettkomponenten in einer Gesamtmenge von bevorzugt 3 bis 20 Gew.-%, besonders bevorzugt 5 bis 15 Gew.-% und insbesondere 6 bis 10 Gew.-%, und weitere hydrophobe Wirkstoffe, wie insbesondere Lichtschutzfilter, in einer Gesamtmenge von 0,5 - 22 Gew.-%, bevorzugt 1 - 15 Gew.-%, besonders bevorzugt 3 - 12 Gew.-%, und außerordentlich bevorzugt 5 - 10 Gew.-% enthalten sind, jeweils bezogen auf das Gesamtgewicht der Pflegeemulsion.

Die Einwaage der obligatorischen Emulgatoren (i) und (ii) wird nicht zur Ölphase gerechnet.

Die Menge der Wasserphase in den erfindungsgemäßen Emulsionen beträgt bevorzugt > 35 Gew.-%, mehr bevorzugt > 45 Gew.-%, besonders bevorzugt > 55 Gew.-% und insbesondere bevorzugt > 65 Gew.-% - jeweils bezogen auf das Gesamtgewicht der Emulsion.

Neben den bereits genannten, zwingenden und fakultativen Komponenten, die zu besonders bevorzugten Ausführungsformen der Pflegeemulsionen des erfindungsgemäßen Systems gehören, enthalten die Pflegeemulsionen zur weiteren Steigerung der Hautpflegenden und - schützenden Wirkung mindestens einen oder mehrere Hilfs- und Wirkstoffe, die ausgewählt sind aus der Gruppe der UV-Lichtschutzfilter, Antioxidantien, Hyaluronsäure sowie deren physiologisch verträglichen Salzen und Derivaten, Wirkstoffen zur Stimulation der Kollagensynthese, Wirkstoffen zur Erhöhung bzw. Verbesserung der Interaktion zwischen der extrazellulären Matrix und den Fibroblasten sowie Wirkstoffen zur Förderung der Elastinproduktion.

Weitere bevorzugte Pflegeemulsionen des erfindungsgemäß Systems sind **dadurch gekennzeichnet, dass** sie zusätzlich zu der zwingenden Wirkstoffkombination nach Anspruch 1 mindestens einen die Kollagensynthese stimulierenden Wirkstoff enthalten, der ausgewählt ist aus
- Retinoiden, insbesondere Retinol und C₂-C₂₂-Fettsäureestern des Retinols,
- Kombucha,
- Olivenblattextrakten (Olea Europaea (Olive) Leaf Extract),
- Oleanolsäure,
- Oleanol,
- Grüntee-Extrakten (Camellia Sinensis),
- Kreatin,
- sowie Mischungen der vorgenannten Substanzen.

Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-cis-Retinsäure und 13-cis-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin*), monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Kollagensynthese stimulieren, sind ausgewählt aus Kombucha.

Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von Mono- oder Disaccharid-haltigem Tee (Schwarzer Tee, Grüner Tee, Kräutertee oder Früchtetee), das ohne Zusatz von Konservierungsstoffen, Farbstoffen und Aromastoffen hergestellt wird. Beim "Kombucha-Teepilz" handelt es sich tatsächlich nicht um einen Pilz, sondern um eine Symbiose von Essigsäurebakterien (meistens *Acetobacter xylinum,* aber auch *Acetobacter gluconicum*) mit verschiedenen Hefen (*Saccharomyces* sp., *Torula* sp., *Pichia fermentans*); fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Minze, Linde (Lindenblüten), Malve, Rotbusch, Kamille usw. Bevorzugte Kombucha ist aus Mono- oder Disaccharid-haltigem Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCI-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Weitere erfindungsgemäß bevorzugte Wirkstoffe, die die Kollagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Kollagensynthese stimulieren, sind ausgewählt aus Oleanolsäure und Oleanol.

Weitere erfindungsgemäß bevorzugte Wirkstoffe, die die Kollagensynthese stimulieren, sind ausgewählt aus Grüntee-Extrakten (Camellia Sinensis). Ein erfindungsgemäß besonders bevorzugter Grüntee-Extrakt ist liposomenverkapselter Grüntee-Extrakt, beispielsweise unter der Handelsbezeichnung Greenselect Phytosome von der Firma Indena erhältlich.

Weitere erfindungsgemäß bevorzugte Wirkstoffe, die die Kollagensynthese stimulieren, sind ausgewählt aus Oligopeptiden und Kreatin sowie aus Mischungen aller vorgenannten Wirkstoffe.

Der oder die Wirkstoff(e), der (die) die Kollagensynthese stimuliert(en), werden in den erfindungsgemäßen Pflegeemulsionen - bezogen auf deren Gesamtgewicht - in einer Menge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, eingesetzt.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen weiteren, die Interaktion zwischen der extrazellulären Matrix und den Fibroblasten erhöhenden und/oder verbessernden Wirkstoff enthalten, der ausgewählt ist aus
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Mischungen aus mindestens einem Extrakt aus Kakaobohnen (Theobroma cacao) und min destens einem Extrakt aus den Blättern der Pfefferminze (Mentha piperita),
- Hydroxystilbenen und deren Estern, insbesondere Resveratrol und/oder Resveratrolmono-, -di- und -triphosphorsäureestern und deren Salzen,
- Isoflavonoiden und Isoflavonoid-reichen Pflanzenextrakten,
- Dihydroquercetin (= Taxifolin),
- sowie Mischungen hiervon.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich. Besonders bevorzugte Pflegeemulsionen des erfindungsgemäßen Systems sind **dadurch gekennzeichnet, dass** sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Apfelkernextrakt in Mengen von 0,001 - 2 Gew.-%, bevorzugt 0,01 - 1,6 Gew.-% und besonders bevorzugt 0,03 - 1 Gew.-%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich. Weitere erfindungsgemäß bevorzugte Wirkstoffe, die die Interaktion zwischen der extrazellulären Matrix und den Fibroblasten erhöhen und/oder verbessern, sind ausgewählt aus Rotweinextrakten (Wine Extract). Ein erfindungsgemäß besonders bevorzugter Rotweinextrakt ist unter der Handelsbezeichnung Sepivinol R von der Firma Seppic erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Traubenkernextrakten (Vitis Vinifera (Grape) Seed Extract), die besonders bevorzugt aus der Chardonnay-Traube stammen. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Mischungen aus mindestens einem Extrakt aus Kakaobohnen (Theobroma cacao) und mindestens einem Extrakt aus den Blättern der Pfefferminze (Mentha piperita). Erfindungsgemäß besonders bevorzugte Mischungen aus mindestens einem Extrakt aus Kakaobohnen (Theobroma cacao) und mindestens einem Extrakt aus den Blättern der Pfefferminze (Mentha piperita) - wobei wässrige, glycolische oder wässrig-glycolische Zubereitungen dieser Extraktmischungen besonders bevorzugt sind - sind unter den Handelsnamen Caomint, Caophenol, Caobromine, Caospice und Caoorange von Solabia erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Hydroxystilbenen und deren Estern, insbesondere Resveratrol (trans-Stilben-3,4'-5-triol) und/oder Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten.

Zu den Isoflavonoiden zählen erfindungsgemäß die Isoflavone und die Isoflavon-Glycoside.

Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Erfindungsgemäß besonders bevorzugte kosmetische oder dermatologische Zusammensetzungen sind **dadurch gekennzeichnet, dass** sie mindestens ein Isoflavonoid in Gesamtmengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Besonders bevorzugte Pflegeemulsionen des erfindungsgemäßen Systems sind **dadurch gekennzeichnet, dass** sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.-%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Als erfindungsgemäß geeigneter Wirkstoff zur Förderung der Elastinproduktion dient ein Dillextrakt (Peucedanum Graveolens Extract), beispielsweise ein unter der Handelsbezeichnung AHYA 050 A (Gemisch aus Wasser, Butylen Glycol, Dillextrakt und Xanthan Gum) erhältliches Produkt. Dillextrakt wird in den erfindungsgemäßen Pflegeemulsionen - bezogen auf deren Gesamtgewicht - in einer Menge von 0,0001 bis 3 Gew.-%, bevorzugt von 0,0005 bis 2 Gew.-% und insbesondere 0,001 bis 1,5 Gew.-% eingesetzt.

Weitere bevorzugte Wirkstoffe, die den erfindungsgemäßen Pflegeemulsionen zusätzlich zu den zwingenden Wirkstoffen nach Anspruch zugesdetzt werden können und den Hautzustand positiv beeinflussen sind ausgewählt aus der Gruppe, die gelildet wird aus:
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden und
- sebumregulierenden Wirkstoffen sowie
- Mischungen dieser Wirkstoffe.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen Wirkstoff enthalten, der ausgewählt ist aus DNA-Oligonucleotiden und RNA-Oligonucleotiden.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).

Ein erfindungsgemäß besonders bevorzugtes Oligonucleotid ist das Thymidin-Dinucleotid.

In den besonders bevorzugten erfindungsgemäßen Pflegeemulsionen sind die DNA-Oligonucleotide oder RNA-Oligonucleotide in Mengen von 0,00001-5 Gew.-%, bevorzugt 0,0001 - 1,0 Gew.-% und besonders bevorzugt 0,0005 - 0,5 Gew.-%, bezogen auf die gesamte Zusammensetzung, enthalten.

Weitere bevorzugte Pflegeemulsionen des erfindungsgemäßen Systems sind dadurch gekennzeichnet, dass sie zusätzlich zu der zwingenden Wirkstoffkombination nach Anspruch 1 mindestens einen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Men gen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotin säureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungs gemäß einsetzbare Derivate des Panthenols sind insbesondere die Ester und Ether des Pan thenols sowie kationisch derivatisierte Panthenole. In einer weiteren bevorzugten Ausführ ungsform der Erfindung können an Stelle von sowie zusätzlich zu Pantothensäure oder Pan thenol auch Derivate des 2-Furanon mit der allgemeinen Strukturformel (VIT-I) eingesetzt werden. 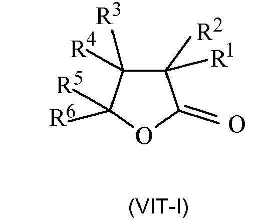 Bevorzugt sind die 2-Furanon-Derivate, in denen die Substituenten R¹ bis R⁶ unabhängig von einander ein Wasserstoffatom, einen Hydroxylrest, einen Methyl-, Methoxy-, Aminomethyl-oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasser stoffrest oder einen gesättigten oder ein- oder zweifach ungesättigten, verzweigten oder line aren Mono-, Di- oder Triamino-C₂-C₄ - Kohlenwasserstoffrest darstellen. Besonders bevor zugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γbutyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3- hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoff atom stehen. Das Stereoisomer (R)-Pantolacton entsteht beim Abbau von Pantothensäure.
   Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin 8₇ (Biotin), auch als Vitamin H oder "Hautvitamin" bezeichnet. Bei Biotin handelt es sich um (3aS,4S, 6aR)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind **dadurch gekennzeichnet, dass** sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind **dadurch gekennzeichnet, dass** sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.

Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer-und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.

Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen Wirkstoff enthalten, der ausgewählt ist aus mindestens einem Flavonoid und/oder mindestens einem Flavonoid-reichen Pflanzenextrakt.

Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-a-L-mannopyranosyl)-β-D-glucopyranosid)), Monoxerutin (3,3',4',5-Tetrahydroxy-7-(2-hydroxyethoxy)flavon-3-(6-O-(6-deoxy-a-L-mannopyranosyl)-β-D-glucopyranosid)), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen Wirkstoff enthalten, der ausgewählt ist aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-1) auf: 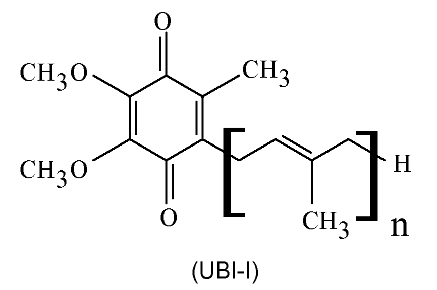 mit n = 6, 7, 8, 9 oder 10.

Besonders bevorzugt ist das Ubichinon der Formel (UBI-1) mit n = 10, bekannt als Coenzym Q10. Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 Silymarin enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens eine anorganische und/oder mindestens eine organische UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4`methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.

Erfindungsgemäß sind die anorganischen UV-Filter bevorzugt in Mengen von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten. Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin.

Erfindungsgemäß sind die selbstbräunenden Wirkstoffe in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten. Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen Hautberuhigenden Wirkstoff enthalten. Erfindungsgemäß bevorzugte Hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter dem Handelsnamen Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, Extrakten aus den Samen von Cucurbita pepo (Zucchini), Extrakten aus Mentha piperita, insbesondere aus den Blättern von Mentha piperita, N-acylierten Alkanolaminen, insbesondere N-Palmitoylethanolamin, Hydrolysaten und/oder Extrakten aus der Alge *Enteromorpha compressa* (Darmtang, eine Grünalge), insbesondere aus dem Vegetationskörper (Thallus) der Alge, kommerziell erhältlich z. B. unter dem Handelsnamen Enteline 2, weiterhin ausgewählt aus dem Dipeptid Tyr-Arg, dessen Estern und dessen N-Acyl-Derivaten, insbesondere N-Acetyl-Tyr-Arg-hexyldecylester (z. B. Calmosensine von Sederma, sowie beliebigen Mischungen dieser Substanzen.

Erfindungsgemäß besonders bevorzugte Extrakte aus den Samen von Cucurbita pepo (Zucchini) sind beispielsweise in den Handelsprodukten Curbilene (ex Indena SpA, INCI: Cucurbita pepo (pumpkin) seed extract), Ocaline (ex Soliance, INCI: Sea Water (and) Water (and) Cucurbita pepo (pumpkin) seed extract), ARP 100 (ex Greentech, INCI: Water, Alcohol, Serenoa Serrulata Fruit Extract, Epilobium Angustifolium Extract, Cucurbita Pepo (Pumpkin) Seed Extract), ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), Cucurbitine (ex Christian Dior Parfums, INCI: Water (and) Cucurbita pepo (pumpkin) seed extract), Herbasol Extract Pumpkin (ex Cosmetochem, INCI: Water (and) Propylene glycol (and) Cucurbita pepo (pumpkin) seed extract) und Pumpkin Extract (ex Draco Natural Products, INCI: Water (and) Cucurbita pepo (pumpkin) seed extract) enthalten.

Erfindungsgemäß besonders bevorzugte Extrakte aus Mentha piperita, insbesondere aus den Blättern von Mentha piperita, sind beispielsweise in den Handelsprodukten Calmiskin OP (ex Silab, INCI: Water (and) Mentha piperita (Peppermint) Leaf Extract) und Caomint (ex Solabia, INCI: Propylene Glycol, Water, Mentha piperita (Peppermint) Leaf Extract, Theobroma Cacao (Cocoa) Extract) enthalten.

Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens einen sebumregulierenden Wirkstoff. Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobiologiques, INCI: Aqua, Hydrolyzed Yeast Protein, Pyridoxine, Niacinamide, Glycerin, Panthenol, , Allantoin, Biotin) und Antifettfaktor^{®} COS-218/2-A (Cosmetochem, INCI: Aqua, Cetyl-PCA, PEG-8 Isolauryl Thioether, PCA, Cetyl Alcohol).

Die sebumregulierenden Wirkstoffe sind bevorzugt in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Weitere bevorzugte Pflegeemulsionen des erfindungsgemäßen Systems sind **dadurch gekennzeichnet, dass** sie zusätzlich zu der zwingenden Wirkstoffkombination nach Anspruch 1 mindestens ein Polysaccharid enthalten, das in einer bevorzugten Ausführungsform ausgewählt ist aus Glycosaminglycanen, besonders bevorzugt aus Hyaluronsaeure, Dextran, Dextransulfat, Chondroitin-4-sulfat und Chondroitin-6-sulfat und insbesondere bevorzugt aus Hyaluronsäure. Das Polysaccharid wird den erfindungsgemäßen Pflegeemulsionen - bezogen auf deren Gesmtgewicht - in einer Menge von 0,0001 bis 2 Gew.-%, bevorzugt von 0,0005 bis 1,5 Gew.-% und insbesondere in Mengen von 0,001 bis 1 Gew.-% zugegeben.

Die Pflegeemulsionen des erfindungsgemäßen Systems weisen einen pH-Wert im hautschonenden Bereich von 3,5 bis 7,5, bevorzugt von 4 bis 7 und insbesondere von 4,5 bis 6,5 auf.

Darüber hinaus können die erfindungsgemäßen Emulsionen fakultativ die weiteren Hilfs-, Zusatz-und Wirkstoffe enthalten:
- Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als desodorierende Wirkstoffe u.a. keimhemmende Mittel, Enzyminhibitoren, Geruchsabsorber oder I Geruchsüberdecker.
- Insekten-Repellentien: Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent 3535 von der Merck KGaA vertrieben wird, sowie Butylacetylaminopropionate in Frage.
- Selbstbräuner: Als Selbstbräuner eignet sich Dihydroxyaceton. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.
- Konservierungsmittel: Als Konservierungsmittel eignen sich beispielsweise Phenoxyethanol, Formaldehydlösung, Parabene, Pentandiol oder Sorbinsäure sowie die unter der Bezeichnung SurfacineG bekannten Silberkomplexe und die in Anlage 6, Teil A und B der Kosmetikverordnung aufgeführten weiteren Stoffklassen.
- Parfümöle: Als Parfümöle seien genannt Gemische aus natürlichen und synthetischen Riechstoffen. Natürliche Riechstoffe sind Extrakte von Blüten, Stängeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.
- Farbstoffe: Als Farbstoffe können die für kosmetische Zwecke geeigneten und zugelassenen Substanzen verwendet werden. Beispiele sind Cochenillerot A (C.I. 16255), Patentblau V (C.1.42051), Indigotin (C.1.73015), Chlorophyllin (C.1.75810), Chinolingelb (C.1.47005), Titandioxid (C.1. 77891), Indanthrenblau RS (C.1. 69800) und Krapplack (C.1.58000). Diese Farbstoffe werden üblicherweise in Konzentrationen von 0,001 bis 0,1 Gew.-%, bezogen auf die gesamte Mischung, eingesetzt.

Die Pflegeemulsionen des erfindungsgemäßen Systems sind insbesondere für die Applikation in einem manuell betätigbaren System mit Pumpmechanismus geeignet.

Besonders bevorzugt ist es, wenn die erfindungsgemäße Pflegeemulsion in einem Schaumspender mit einem Pumpmechanismus zur Verschäumung mit Luft enthalten ist. Ein derartiger Schaumspender ermöglicht es, auf Treibgase zu verzichten. Besonders vorteilhaft bei diesem Typ von Spendern ist auch, dass die Anwendung besonders einfach ist, der entstehende Schaum eine leichte und cremige Textur, keine Drainage sowie eine einfache Verteilbarkeit aufweist, wodurch alle vorgenannten positiven Effekte auf der Haut erzielt werden können. Erfindungsgemäß besonders geeignete Schaumspender werden in den Anmeldungen WO 2003/088941 und WO 2004/078902 ausführlich beschrieben. Auf diese Anmeldungen wird hinsichtlich der Beschreibung des Pumpspenders daher ausdrücklich Bezug genommen.

Diese für die erfindungsgemäßen Pflegeemulsionen besonders geeigneten Schaumspender sind im Handel unter den Bezeichnungen M3 und F3 von der Firma Airspray erhältlich.

Um unter Verwendung eines manuellen Pumpspenders aufschäumbar zu sein, sind bevorzugte erfindungsgemäße Zusammensetzungen gekennzeichnet durch eine Viskosität bei 21 °C im Bereich von 50 - 1500 mPas, bevorzugt 100 - 1000 mPas, besonders bevorzugt 400 - 850 mPas, außerordentlich bevorzugt 500 - 750 mPas, gemessen mit einem Brookfield Rotationsviskosimeter, Spindel Nr. 1 bei einer Rotationsgeschwindigkeit von 20 UpM oder mit einer Spindel T-A bei einer Rotationsgeschwindigkeit von 12 UpM. Die folgenden Beispiele sollen den Gegenstand der Erfindung näher beschreiben, ohne ihn darauf einzuschränken. Die Mengenangaben beziehen sich dabei - sofern nicht anders angegeben - auf Gew.-%.

### Beispiele:

### 1) Schäumende Pflegeemulsion

| **Handelsname** | **INCI-Bezeichnung und Hersteller** | **Einsatzmenge** |
|---|---|---|
| **ABIL® EM 90** | Cetyl PEG/PPG-10/1-Dimethicone (Evonik Degussa) | 5,0 |
| **Cetiol® OE** | Dicaprylyl Ether (Cognis) | 2,0 |
| **Finsolv®TN** | C12-C15-Alkylbenzoat (Innospec) | 3,0 |
| **Parsol® SLX** | Benzylidene Malonate Polysiloxane (DSM) | 3,0 |
| **Neo Heliopan® 303** | Octocrylene (Symrise) | 4,0 |
| **Parsol® 1789** | Butyl Methoxydibenzoylmethane (DSM) | 2,0 |
| **Tocopheryl Acetat** | Tocopheryl Acetat | 0,6 |
| **Sepinov® EMT 10** | Hydroxyethyl Acrylate/Sodium-Acryloyldimethyl Taurate Copolymer (Seppic) | 0,1 |
| **Glycerin, 86%** | Glycerin | 3,0 |
| **Phenonip® ME** | Phenoxyethanol, Methylparaben, Ethylparaben (Clariant) | 1,0 |
| **Texapon® SB 3 unkons.** | Disodium Laureth Sulfosuccinate (Cognis) | 3,0 |
| **Tego Betain® 810** | Capryl/Capramidopropyl Betaine (Goldschmidt) | 2,0 |
| **Citronensäure, 10%ig** | Citronensäure | q.s. |

Die Fett- und die Wasserphase werden getrennt auf ca. 80°C erhitzt. Anschließend wird die Fettphase vorgelegt, und unter Verwendung einer Dissolverscheibe mit der Wasserphase emulgiert. Bei einer Temperatur von 30°C wird das Gemisch der tensidischen Co-Emulgatoren eingearbeitet. Nach einer Nachrührzeit von 20 Minuten wird die Emulsion mit Citronensäure auf einen pH-Wert von 5,1 eingestellt.

Die Emulsion wird in einem Foamer des Modells F3 oder M3 der Firma Airspray konfektioniert. Der jeweilige Pflegeschaum kann auf diese Weise einfach und in der richtigen Dosierung für die Anwendung entnommen werden. Der Schaum ist besonders cremig und feinporig und lässt sich leicht auf der Haut verteilen, wobei er ein konstant gleitendes Pflegegefühl hinterlässt.

## Patentansprüche

1. System aus einem manuell anwendbaren Schaumspender und einer kosmetischen Pflegeemulsion, **dadurch gekennzeichnet, dass** die Emulsion
(a) mindestens einen hautpflegenden Ölkörper,
(b) ein Tensidgemisch (i) + (ii) aus
(i) mindestens einem milden anionischen Tensid und
(ii) mindestens einem milden amphoteren und/oder zwitterionischen Tensid und
(c) einen Silikonemulgator mit einem HLB-Wert im Bereich von 4 bis 6, enthält.

2. System nach Anspruch 1, **dadurch gekennzeichnet, dass** das oder die Tenside (i) der Pflegeemulsion ausgewählt sind aus einem oder mehreren Tensiden der Gruppe der Acylaminosäuren, alpha-Olefinsulfonate, ethoxylierten Alkylschwefelsäurester, Sulfobernsteinsäureester, Ethercarbonsäuren und/oder den Salzen dieser Verbindungen.

3. System nach einem der Ansprüche 1 oder 2, **dadurch gekennzeichnet, dass** das oder die Tenside (ii) der Pflegeemulsion ausgewählt sind aus einem oder mehreren Tensiden der Gruppe der Alkylbetaine, Alkylamidoalkylbetaine, Aminopropionate, Aminoglycinate, Sulfobetaine, Imidazoliniumbetaine und/oder den Salzen dieser Verbindungen.

4. System nach einem der Ansprüche 1 bis 3, **dadurch gekennzeichnet, dass** der Gehalt an anionischen Tensiden (i) 0,1 bis 20 Gew.-%, bevorzugt 0,2 bis 15 Gew.-%, mehr bevorzugt 0,3 bis 12 Gew.-% und insbesondere 0,5 bis 9 Gew.-% - bezogen auf das Gesamtgewicht der Pflegeemulsion, beträgt.

5. System nach einem der Ansprüche 1 bis 4, **dadurch gekennzeichnet, dass** der Gehalt an amphoteren und/oder zwitterionischen Tensiden (ii) 0,1 bis 20 Gew.-%, bevorzugt 0,2 bis 15 Gew.-%, mehr bevorzugt 0,3 bis 12 Gew.-% und insbesondere 0,5 bis 9 Gew.-% - bezogen auf das Gesamtgewicht der Pflegeemulsion, beträgt.

6. System nach einem der Ansprüche 1 bis 5, **dadurch gekennzeichnet, dass** die Pflegeemulsion mindestens einen Feuchtigkeit spendenden Wirkstoff enthält.

7. System nach einem der Ansprüche 1 bis 6, **dadurch gekennzeichnet, dass** die Pflegeemulsion - bezogen auf ihr Gesamtgewicht - 0,2 bis 20 Gew.-%, bevorzugt 0,5 bis 15 Gew.-%, mehr bevorzugt 1,0 bis 12 Gew.-% und insbesondere 1,5 bis 10 Gew.-% des Silikonemulgators (c) enthält.

8. System nach einem der Ansprüche 1 bis 7, **dadurch gekennzeichnet, dass** die Pflegeemulsion als Silikonemulgator (c) ein oder mehrere Organopolysiloxan-Polyoxyalkylen(e) enthält.

9. System nach einem der Ansprüche 1 bis 7, **dadurch gekennzeichnet, dass** die Pflegeemulsion zur Erhöhung der Emulsions- und Schaumstabilität eine Kombination aus mindestens zwei modifizierten Polymeren enthält, von denen eines natürlichen und das andere synthetischen Ursprungs ist.

10. System nach Anspruch 9, **dadurch gekennzeichnet, dass** die Polymere synthetischen Ursprungs ausgewählt sind aus Homo- und Copolymeren der Acrylsäure mit Acrylsäurestern und/oder Acrylamiden und/oder aus Homo- und Copolymeren der Acryloyldimethyltaurate mit Acrylamiden und/oder Alkylacrylaten und die Polymere natürlichen Ursprungs ausgewählt sind aus Xanthan Gum, Guar Gum, Alginaten und/oder Carrageenaten.

11. System nach einem der Ansprüche 1 bis 10, **dadurch gekennzeichnet, dass** die Pflegeemulsion neben einem kosmetisch akzeptablen Ölkörper (a) mindestens einen weiteren kosmetischen Hilfs- oder Wirkstoff enthält, der ausgewählt ist aus der Gruppe der Sonnenschutzmittel, Antioxidantien, Hyaluronsäure sowie deren physiologisch verträglichen Salzen und Derivaten, Wirkstoffen zur Stimulierung der Kollagensynthese, Wirkstoffen zur Erhöhung bzw. Verbesserung der Interaktion zwischen der extrazellulären Matrix und den Fibroblasten sowie Wirkstoffen zur Förderung der Elastinproduktion.

12. System nach einem der Ansprüche 1 bis 11, **dadurch gekennzeichnet, dass** die Pflegeemulsion einen pH-Wert im Bereich von 3,5 bis 7,5, bevorzugt von 4 bis 7 und insbesondere von 4,5 bis 6,5 aufweist.

13. System nach einem der Ansprüche 1 bis 12, **dadurch gekennzeichnet, dass** die Pflegeemulsion in einem Schaumspender mit einem Pumpmechanismus zur Verschäumung mit Luft enthalten ist.

14. Verwendung eines Silikonemulgators eines HLB-Werts von 4 bis 6 in kosmetischen Pflegeemulsionen, die in einem manuell anwendbaren Schaumspender konfektioniert sind.

15. Verwendung eines Systems nach einem der Ansprüche 1 bis 13 zur Pflege von Keratinfasern, insbesondere der menschlichen Haut.
